# EUROPEAN PATENT SPECIFICATION

(11) **EP 0 768 881 B1**
(45) Date of publication and mention of the grant of the patent: **12.06.2002**
(21) Application number: 95924930.1
(22) Date of filing: 23.06.1995
(51) Int. Cl.: A61K 31/505, A61K 31/70, A61K 31/165, A61P 31/18

(54) **ANTI-HIV TRIPLE COMBINATION COMPRISING ZIDOVUDINE, LAMIVUDINE AND LOVIRIDE**
Anti HIV - Dreifachkombination enthaltend Zidovudine, Lamivudine und Loviride
TRIPLE COMBINAISON ANTI-VIH COMPRENANT LA ZIDOVUDINE, LA LAMIVUDINE ET LE LOVIRIDE

(30) Priority: 01.07.1994 EP 94201897
(43) Date of publication of application: 23.04.1997
(73) Proprietor: JANSSEN PHARMACEUTICA N.V., 2340 Beerse (BE)
(72) Inventor: STOFFELS, Paulus, Aloysius, Maria, B-2320 Hoogstraten (BE); ANDRIES, Koenraad, Jozef, Lodewijk, Marcel, B-2340 Beerse (BE)
(86) International application number: EP9502462
(87) International publication number: WO9601110

(56) References cited:
- EP-A- 0 513 917
- Biosis Abstracts accession no. 98025452 & Abstracts of the Interscience Conference on Antimicrobial Agents and Chemotherapy 34 (0). 1994. 133 (Oct. 4-7, 1994)
- NATURE, vol. 365, 30 September 1993 pages 451-453, BRENDAN L. LARDER ET AL. 'Convergent combination therapy can select viable multidrug-resistant HIV-1 in vitro'
- INFECTIOUS AGENTS AND DISEASE, vol. 2, no. 5, 1993 pages 291-303, CARLA B. PETTINELLI ET AL. 'Treatment Research Priorities for Human Immunodeficiency Virus Infection'
- INFECTIOUS AGENTS AND DISEASE, vol. 3, no. 6, 1994 pages 313-323, J.MICHAEL KILBY ET AL. 'Is There a Role for Non-nucleoside Reverse Transcriptase Inhibitors in the Treatment of HIV Infection?'
- EXP.OPIN.INVEST.DRUGS, vol. 3, no. 3, 1994 pages 253-271, ERIK DE CLERCQ 'Non-nucleoside reverse transcriptase inhibitors (NNRTIs)'

## Description

The present invention relates to a therapeutic combination comprising zidovudine, lamivudine and loviride for the treatment of Human Immunodeficiency Virus (HIV) infections and especially for the treatment of AIDS or AIDS-related complex (ARC).

It is now 13 years since Acquired Immunodeficiency Syndrome (AIDS) sprang into medical and public awareness as a fatal disease of the immune system. More than three million people have developed full-blown AIDS; most have already died. Responsible estimates of the number of cases of HIV infection likely to have occurred by the year 2000 range from 40 million to more than 110 million.
It is now generally accepted that the Human Immunodeficiency Virus (HIV) is the aetiological agent of AIDS and AIDS-related complex (ARC). Not only is it believed that HIV is the primary cause of AIDS but also that HIV-infection alone will usually cause profound immune dysfunction over time with lethal consequences for the patient. Due to public attention, AIDS has become one of the most intensively studied diseases and HIV has become the most intensively studied virus in history. In spite of all this scientific effort, still there is no effective cure or even satisfactory treatment for this life threatening condition.
The antiviral drugs that have been approved in the U.S.A. for treatment of HIV infection, i.e. 3'-azido-3'-deoxythymidine (AZT), dideoxycytidine (ddC) and dideoxyinosine (ddI), all work by interfering with the reverse transcription. However, HIV shows a high rate of mutation and within months variant reverse transcriptases appear in the body that can produce viral DNA even in the presence of said reverse transciptase inhibitors. In other words HIV becomes resistant to these drugs. This resistance, as well as the toxicity of the nucleoside type reverse transcriptase inhibitors explain why the benefits of drugs like AZT are only temporary.
"Convergent triple therapy" has been devised to circumvent this problem of resistance. One study showed that a combination of zidovudine, dideoxyinosine and one other drug, nevirapine or pyridinone, produced *in vitro* viral isolates incompatible with HIV replication (Chow Y-K et al, Nature, volume 361, (1993)). However, the results of this experiments are incorrect because also unintended mutations were discovered in the reverse transcriptase inhibitor of the non-viable virus (Chow Y-K et al, Nature, volume 364 (1993)).

WO 93/23021 describes therapeutic combinations for the treatment of HIV-infections comprising zidovudine and an agent serving to enhance the antiviral activity of zidovudine against HIV populations otherwise resistant to zidovudine. This patent application shows that zidovudine resistant viruses could be made zidovudine sensitive again by exposing *in vitro* said viruses to "chloro-TIBO" (9-chloro-4,5,6,7-tetrahydro-5-methyl-6-(3-methyl-2-butenyl)-5-imidazo[4,5,i-jk] benzodiazepine-2(1H)-thione), or FTC ((-)-2',3'-dideoxy-5-fluoro-3'-thiacytidine) and lamivudine.

EP-A-0,513,917 describes double combinations of lamivudine and an inhibitor of HIV replication. Said patent application encompasses *in vitro* data showing that there is some synergistic anti-HIV activity when using combinations of lamivudine together with zidovudine, dideoxyinosine, Ro 31-8539 (N'-benzyl-3[4a(S),8a(S)-3(S)-(tert-butylcarbamoyl)decahydroisoquinolin-2-yl]-2(R)-hydroxypropyl]-N"-(quinolin-2-yl-carbamoyl)-L-asparaginamide), or TIBO ((+)-S-4,5,6,7-tetrahydro-5-methyl-6-(3-methyl-2-butenyl)imidazo(4,5,1-jk)(1,4)-benzodiazepin-2-(1H)thione).

The problem to be solved is to find a combination of compatible agents which shows efficacy against HIV-infection *in vivo* and which is tolerable by HIV-infected patients. The term "compatible" means that one agent (called hereinafter "component") does not act negatively on the pharmacokinetic profile of another agent.

Unexpectedly we have found that the triple combination comprising zidovudine, lamivudine and loviride shows synergistic activity in important clinical "surrogate marker" tests, i.e. the CD4 cell count and the p24 antigen count. Moreover the combination is well tolerated by the patients and the components are compatible with each other. What is even more surprising is that the clinical situation of patients receiving the triple combination improved dramatically.

The components of the present triple combination are all known in the art.

Zidovudine, which has the chemical name 3'-azido-3'-deoxythymidine (AZT), is now well established as a useful chemotherapeutic agent for the treatment of HIV-infections. Zidovudine is also used for the treatment of patients who have an asymptomatic HIV infection or who are anti-HIV-antibody-positive. Zidovudine can be prepared, for example, as described in US 4,724,232.

Lamivudine, which has the chemical name (-)-2',3'-dideoxy-3'-thiacytidine (3TC), is described in European Patent Specification No. 0,382,526. Lamivudine is an antiviral nucleoside analogue containing an oxathiolane residue in place of the sugar residue.

Loviride, which has the chemical name (±)-α-[(2-acetyl-5-methylphenyl)amino]-2,6-dichlorobenzeneacetamide is described in European Patent Application EP-A-0,538,301. Loviride is an antiviral non-nucleoside reverse transcriptase inhibitor of the α-anilinophenylacetamide (α-APA) type.

As already mentioned above, it was unexpectedly found that the combination of these three components shows a remarkable synergistic anti-HIV activity *in vivo.*

A first remarkable feature is that the median CD4 cell count increased up to almost 60 % from baseline and remains at more than 25 % above baseline for at least 5 months. (Baseline is the number of CD4-count at the start of the therapy.) The CD4 count of patients receiving established anti-HIV drugs like zidovudine or lamivudine normally shows an increase of about 20% and then said CD4 count decreases again. Even patients receiving a double combination of zidovudine and loviride only show a CD4 increase of about 30% which declines after some weeks.

The CD4 cell count is an important biomedical parameter, a so-called "surrogate marker" (not to be confused with markers which are present in the cell membrane), for determining the condition of the immune system of an HIV infected patient and which predicts the clinical condition of said patient. On and in the cell membrane of T-lymphocytes there are several markers present. Those markers, which are mostly glycoproteins, allow for identification of the T-lymphocytes. For most of these markers monoclonal antibodies have been prepared which allow modern biomedical techniques to detect cells having these markers. The monoclonal antibody OKT4 recognizes T-helper/inducer cells. According to the "Clusters of Differentiation" (CD) nomenclature the marker recognized by the OKT4 antibody is called CD4 marker, cells having this marker are called CD4 cells. T-lymphocytes with CD4 markers are also called T4-cells. The CD4 cell count is important because the CD4 cells and especially the T4-cells are infected and impaired by HIV. Indeed, the coat of the HIV-virion (a HIV-virus particle) bears a viral protein "spike" that can bind tightly to the above described CD4 markers.
This property makes such CD4 bearing cells vulnerable to HIV-infection. When the "spike" of a virion binds to a CD4 bearing cell, the membranes of the virus and the cell fuse. The virus core and its contents are then brought inside the cell. Once an HIV virion has entered a cell, a complex sequence of events follows that, if completed, leads to the budding of new virus particles from the infected cell. Through different cytopathic mechanisms CD4 cells are progressively killed or destroyed in the course of HIV infection. The loss of CD4 cells is the primary course of the extensive impairment of the immune system. Hence, the amount of CD4 cells is an important parameter for the condition of the immune system.

Another "surrogate marker" used to measure the stage of the infection is to measure the amount of p24 antigen. p24 is a core protein of the HIV-virion. Hence measuring the amount of p24 antigen in plasma gives an estimate of the viral load, i.e. the number of viruses present in the body.

However, the only true marker for the patient is, of course, his clinical condition and his well being. Although this parameter is very hard to quantify the results shown hereinunder are convincing enough to support the present invention.

Examples of viral infections and associated clinical conditions which can be treated or prevented with the invention, include human retroviral infections such as HIV, e.g. HIV-1 or HIV-2, and human T-cell lymphotropic virus (HTLV), e.g. HTLV-I or HTLV-II-infections. The combination of the present invention is especially useful for the treatment of AIDS and related clinical conditions such as AIDS-related complex (ARC), progressive generalized lymphadenopathy (PGL), AIDS-related neurological conditions, such as multiple sclerosis. The combination of the present invention may be particularly applicable to the treatment of asymptomatic infections, primary infections or diseases caused by or associated with HIV.

It will be appreciated that in accordance with the present invention the components of the combination may be administered simultaneously, concurrently or sequentially.

The compounds employed in accordance with the present invention may be administered to patients in a conventional manner. As indicated above, the components of the above combinations may be administered simultaneously (e.g. in a unitary pharmaceutical formulation) or separately (e.g. in separate pharmaceutical formulations). In general, the combinations may be administered by topical, oral, rectal or parenteral (e.g. intravenous, subcutaneous or intramuscular) routes. It will be appreciated that the route may vary with, for example, the severity of the condition of the patient to be treated.

The respective doses of the components are in the range of 0.5 to 120 mg/kg per day, preferably in the range of 1 to 90 mg/kg and most preferably in the range of 3 to 20 mg/kg per day. The desired dose is preferably presented as two, three, four, five, six or more sub-doses administered at appropriate intervals throughout the day. These subdoses may be administered in unit dose forms, for example, containing a total of 10 to 1500 mg, preferably 20 to 1000 mg, and most preferably 50 to 700 mg of component per unit dose form.

The preferred regime is 200 mg zidovudine t.i.d., lamivudine 300 mg b.i.d. and loviride 100 mg t.i.d.

The weight ratio of each couple of components of the triple combination taken on a daily basis may vary in a range from 1/10 to 10/1. More interestingly the weight ratio of each couple varies between 1/2 and 2/1.

The weight ratio zidovudine/lamivudine/loviride is preferably 1/1/0.5.

### Experimental part

The triple combination of zidovudine (200 mg t.i.d.), lamivudine (300 mg b.i.d.) and loviride (100 mg t.i.d.) versus the double combination of zidovudine (200 mg t.i.d.) and loviride (100 mg t.i.d.) was assessed in an open case-control study. Twenty HIV-1 infected patients with a positive p24 antigen test at baseline were included.
Both combinations were well tolerated. Serious drug-induced safety-related abnormalities were not observed. No clinically relevant pharmacokinetic interaction was noted between lamivudine and zidovudine or loviride.
The median CD4 count (% versus baseline) during therapy in the group of patients treated with the triple combination were compared with the median CD4 count in the group of patients treated with the double combination in Table 1.
The median p24 antigen count (% versus baseline) of the group of patients treated with the triple combination were compared with the median p24 antigen count of the group of patients treated with double combination in Table 2.

### Clinical evaluation

Ten patients started the triple combination therapy. Nine patients on the triple combination therapy completed 20 weeks of treatment. One patient dropped out of the study because of an intercurrent infection.

Seven out of 10 patients who received the triple combination therapy improved clinically. After treatment, several patients with ARC symptoms and a poor clinical condition at baseline felt clinically very well, started working or travelling again. The two other patients maintained a stable clinical condition.

**Table 1**

| | CD4 count (% versus baseline) | |
|---|---|---|
| | triple combination | double combination |
| start of therapy | 100 % | 100 % |
| month 1 | 153 % | 132 % |
| month 2 | 143 % | 93 % |
| month 3 | 148 % | 95 % |
| month 4 | 144 % | 96 % |

**Table 2**

| | p24 antigen count (% versus baseline) | |
|---|---|---|
| | triple combination | double combination |
| start of therapy | 100 % | 100 % |
| month 1 | 22 % | 56 % |
| month 2 | 18 % | 72 % |
| month 3 | 33 % | 79 % |
| month 4 | 57 % | 79 % |

## Claims

1. A combination comprising zidovudine, lamivudine and loviride.

2. A combination as claimed in claim 1 wherein the weight ratio zidovudine/lamivudine/loviride is about 1/1/0.5.

3. A composition comprising a pharmaceutically acceptable carrier and as an active ingredient an effective amount of zidovudine, lamivudine and loviride.

4. A composition as claimed in claim 3 wherein the weight ratio of zidovudine/lamivudine/loviride is about 1/1/0.5.

5. A process of preparing a composition as claimed in claims 3 or 4 wherein a pharmaceutically acceptable carrier is intimately mixed with zidovudine, lamivudine and loviride.

6. A combination as claimed in claim 1 for use as a medicine.

7. Use of a combination as claimed in claim 1 for the manufacture of a medicament for the treatment of patients suffering from HIV-infection.

8. A product comprising zidovudine, lamivudine and loviride as a combined preparation for simultaneous, separate or sequential use in anti-HIV treatment.

## Patentansprüche

1. Zusammensetzung mit Zidovudin, Lamivudin und Lovirid.

2. Kombination nach Anspruch 1, in der das Gewichtsverhältnis Zidovudin/Lamivudin/Lovirid ungefähr 1/1/0,5 beträgt.

3. Zusammensetzung mit einem pharmazeutisch unbedenklichen Träger sowie einer wirksamen Menge an Zidovudin, Lamivudin und Lovirid als Wirkstoff.

4. Zusammensetzung nach Anspruch 3, in der das Gewichtsverhältnis von Zidovudin/Lamivudin/Lovirid ungefähr 1/1/0,5 beträgt.

5. Verfahren zur Herstellung einer Zusammensetzung nach Anspruch 3 oder 4, bei dem man einen pharmazeutisch unbedenklichen Träger innig mit Zidovudin, Lamivudin und Lovirid vermischt.

6. Kombination nach Anspruch 1 als Arzneimittel.

7. Verwendung einer Kombination nach Anspruch 1 zur Herstellung eines Arzneimittels zur Behandlung von Patienten, die an HIV-Infektion leiden.

8. Produkt enthaltend Zidovudin, Lamivudin und Lovirid als Kombinationspräparat zur gleichzeitigen, separaten oder aufeinanderfolgenden Verwendung bei der Behandlung von HIV.

## Revendications

1. Combinaison contenant de la zidovudine, de la lamivudine et du loviride.

2. Combinaison selon la revendication 1, dans laquelle le rapport pondéral de zidovudine/lamivudine/loviride est d'environ 1/1/0,5.

3. Composition contenant un excipient pharmaceutiquement acceptable et une quantité efficace de zidovudine, de lamivudine et de loviride en tant qu'ingrédient actif.

4. Composition selon la revendication 3 dans laquelle le rapport pondéral de zidovudine/lamivudine/loviride est d'environ 1/1/0,5.

5. Procédé de préparation d'une composition selon les revendications 3 ou 4 dans lequel un excipient pharmaceutiquement acceptable est mélangé intimement avec de la zidovudine, de la lamivudine et du loviride.

6. Combinaison selon la revendication 1 pour une utilisation en tant que médicament.

7. Utilisation d'une combinaison selon la revendication 1 pour la fabrication d'un médicament pour le traitement de patients souffrant d'une infection par le VIH.

8. Produit contenant de la zidovudine, de la lamivudine et du loviride en tant que préparation combinée pour une utilisation simultanée, séparée ou séquentielle dans un traitement anti-VIH.
